(19) Europäisches Patentamt
European Patent Office
Office européen des brevets

(11) Veröffentlichungsnummer: **0 014 434**
**B1**

(12) # EUROPÄISCHE PATENTSCHRIFT

(45) Veröffentlichungstag der Patentschrift: **18.05.83**

(21) Anmeldenummer: **80100473.0**

(22) Anmeldetag: **30.01.80**

(51) Int. Cl.³: **C 07 C 91/16,** A 61 K 31/13 // C07C101/30, C07C103/127, C07D263/04

(54) **Neues rechts-drehendes, basisches Derivat des 9,10-Aethano-anthracens, Verfahren zu dessen Herstellung und dieses enthaltende pharmazeutische Präparate.**

(30) Priorität: **02.02.79 CH 1047/79**
**08.01.80 CH 94/80**

(43) Veröffentlichungstag der Anmeldung: **20.08.80 Patentblatt 80/17**

(45) Bekanntmachung des Hinweises auf die Patenterteilung: **18.05.83 Patentblatt 83/20**

(84) Benannte Vertragsstaaten: **AT BE CH DE FR GB IT LU NL SE**

(56) Entgegenhaltungen: **US-A-4 017 542**

(73) Patentinhaber: **CIBA-GEIGY AG, Patentabteilung Postfach, CH-4002 Basel (CH)**

(72) Erfinder: **Storni, Angelo, Dr., Im Feuerbusch 3, CH-4310 Rheinfelden (CH)**

(74) Vertreter: **Zumstein, Fritz sen., Dr., Bräuhausstrasse 4, D-8000 München 2 (DE)**

EP 0 014 434 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neues rechts-drehendes, basisches Derivat des 9,10-Äthanoanthracens, Verfahren zu dessen Herstellung und dieses enthaltende pharmazeutische Präparate

Die vorliegende Erfindung betrifft ein neues rechts-drehendes, basisches Derivat des 9,10-Äthanoanthracens und dessen Säureadditionssalze, Verfahren zur Herstellung dieser Stoffe und pharmazeutische Präparate, welche diese Stoffe enthalten.

Die erfindungsgemässe Verbindung ist das S-(+)-α-[(Methylamino)methyl]-9,10-äthanoanthracen-9(10H)-äthanol der Formel I

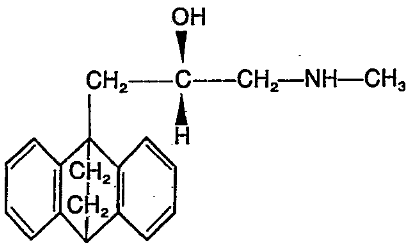

(I)

der (+)-Antipode des in der US-PS 4 017 542 und entsprechenden Patentschriften anderer Länder mit schweizerischer Priorität vom 23. 2. 1971 beschriebenen racemischen α-[(Methylamino)methyl]-9,10-äthanoanthracen-9(10H)-äthanol. Säureadditionssalze der Verbindung der Formel I sind insbesondere pharmazeutisch annehmbare Salze, wie das Hydrobromid, Phosphat, Methansulfonat, Äthansulfonat, 2-Hydroxyäthansulfonat, Acetat, Lactat, Malonat, Succinat, Fumarat, Maleinat, Malat, Tartrat, Citrat, Benzoat, Salicylat, Phenylacetat, Mandelat oder Embonat und vor allem das Hydrochlorid, sowie ganz allgemein gut kristallisierende Salze mit optisch aktiven Säuren, neben den bereits genannten z.B. auch das (1:1)-Salz mit der Bis-0,0′-(p-toluoyl)-D-weinsäure. Infolge der engen Beziehungen zwischen der freien Base und ihren Säureadditionssalzen werden unter der Base und unter ihren Säureadditionssalzen sinngemäss auch die Säureadditionssalze bzw. die freie Base verstanden.

Das S-(+)-α-[(Methylamino)-methyl]-9,10-äthanoanthracen-9(10H)-äthanol und seine Säureadditionssalze besitzen wertvolle pharmakologische Eigenschaften. Vor allem zeigen sie sehr starke Wirkungen in Tests, die für Antidepressiva charakteristisch sind. Eine deutliche Antagonisierung der durch 2 mg/kg sc. Reserpin induzierten Hypothermie an männlichen Mäusen [Askew, Life Sci. 10, 725 (1963), und Benz und Waser, Dissertation 1971, Pharmakolog. Institut der Universität Zürich] ist bereits in Dosen ab 0,3 mg/kg des Hydrochlorids der Verbindung der Formel I erkennbar und ist stärker als die Wirkung von mehrfachen Dosen des entsprechenden Racemats. Die durch 2 mg/kg sec. Reserpin induzierte Ptosis an männlichen Ratten [Rubin et al, J. Pharmacol. Exp. Therap. 120, 125 (1957)] wird durch 0,1 mg/kg p.o. des Hydrochlorids der Verbindung der Formel I bereits deutlich vermindert und durch 0,3 mg/kg p.o. aufgehoben, während das entsprechende, an sich ebenfalls hochwirksame Racemat erst in einer erheblich höheren Dosierung die Ptosis völlig aufhebt.

Neurobiochemisch zeigt das Hydrochlorid der Verbindung der Formel I eine starke Hemmung der Noradrenalin-Aufnahme ins Rattenherz gemäss der Methode von L. Maître, M. Staehelin und H. Bein, Biochem. Pharmacol. 20, 2169 (1971) mit einer $ED_{50}$ von etwa 0,3 mg/kg p.o., während die $ED_{50}$ des entsprechenden Racemats bei etwa 1,5 mg/kg p.o. liegt, wie auch eine starke Hemmung des Noradrenalindepletierenden Effektes von H 77/77 (3-Hydroxy-4,α-dimethylphenäthylamin) im Rattenhirn als indirekte Anzeige der Noradrenalin-Aufnahme-Hemmung [A. Carlsson, H. Corrodi, K. Fuxe und T. Hoefkelt, Europ. J. Pharmacol. 5, 367 (1969)] mit einer $ED_{50}$ von etwa 2 mg/kg p.o., gegenüber einer $ED_{50}$ von etwa 10 mg/kg p.o. des entsprechenden Racemats. Die endogenen Konzentrationen von Noradrenalin und von Dopamin im Rattenhirn, gemessen nach P. Waldmeier, de Herdt und L. Maître [Chir. Chem. 20, 81 (1974)] werden weder vom Hydrochlorid der Verbindung der Formel I in Dosen bis 100 mg/kg bzw. 30 mg/kg p.o., noch vom entsprechenden Racemat in Dosen bis 10 mg/kg bzw. bis 100 mg/kg beeinflusst.

Die Befunde betreffend die Noradrenalin-Aufnahme legen nahe, dass auch die starken Reserpin-antagonistischen Wirkungen der Verbindung der Formel I bzw. ihrer Säureadditionssalze auf einer entsprechenden Beeinflussung von Transport und Metabolismus des Noradrenalins beruhen. Die Wirkungssteigerung auf weit mehr als das Doppelte in diesen Tests lässt sich jedenfalls nicht dadurch erklären, dass allein der im bekannten Racemat vorliegende (+)-Antipode wirksam ist, und ist deshalb als überraschend zu erachten.

In einer Reihe von weiteren Tests ist die Verbindung der Formel I, als Hydrochlorid getestet, ungefähr gleich bis höchstens doppelt so potent wie das entsprechende Racemat. Gleiche Dosen beider Stoffe ergeben ungefähr dieselben Wirkungen, z.B. bei der Antagonisierung der Histamin-Toxizität am Meerschweinchen, bei der Prüfung der exploratorischen Aktivität der Maus [A. Delini-Stula und R. Meier, Neuropharmacology, 15, 383–388 (1976)], und bei der durch Fuss-Schock induzierten Kampfreaktion der Maus [Tedeschi et al., J. Pharmacol. Exptl. Therap. 125, 28–34 (1959)], während bei der Antagonisierung der durch Tetrabenazin induzierten Katalepsie der Ratte nach oraler Verabreichung, und bei der Potenzierung der durch G 29 505 [2-(4-Allyl-2-methoxyphenoxy)-N,N-diäthyl-acetamid] bewirkten Narkose der Maus [W. Theobald und R. Domenjoz, Arzneimittelforsch. 9, 285–286 (1959)] nach intraperitonealer Verabreichung das Hydrochlorid der Verbindung der Formel I bereits in ungefähr der halben Dosierung gleich wirksam ist wie das entsprechende Racemat.

Die akuten Toxizitäten der Verbindung der Formel I und des entsprechenden Racemates bei

oraler und intravenöser Verabreichung sind ungefähr gleich. Gleiche negativ inotrope Wirkungen am isolierten linken Vorhof von reserpinierten, d.h. mit Reserpin vorbehandelten Meerschweinchen wurden durch die Verbindung der Formel I bei ungefähr halb so hohen Konzentrationen wie durch das Racemat bewirkt, während am isolierten rechten Vorhof die negativ chronotrope Wirkung der Verbindung der Formel I schwächer war als diejenige des Racemats, und der Vergleich der Wirkung von je 1 mg/ml beider Stoffe an isolierten Vorhöfen von nichtreserpinierten und von reserpierten Meerschweinchen auf eine ungefähr gleiche kardiostimulierende Wirksamkeit schliessen lässt.

Aus den genannten und weiteren Versuchen ergibt sich, dass die bedeutende Steigerung der Reserpin-antagonistischen Wirksamkeit und der Noradrenalin-Aufnahmehemmung sowie die annähernd doppelte Tetrabenazin-antagonistische Wirksamkeit der Verbindung der Formel I und ihrer Säureadditionssalze, als für Antidepressiva besonders charakteristische Wirkungsqualitäten, im Vergleich zum Racemat und seinen Säureadditionssalzen keineswegs von einer ähnlichen Steigerung der Toxizität und/oder der Nebenwirkungen begleitet ist. Das S-(+)-α-[(Methylamino)-methyl]-9,10-äthanoanthracen-9(10H)-äthanol der Formel I und seine pharmazeutisch annehmbaren Salze können als Antidepressiva, insbesondere zur Behandlung von Gemütsdepressionen, Verwendung finden.

Das S-(+)-α-[(Methylamino)methyl]-9,10-äthano-anthracen-9(10H)-äthanol der Formel I und seine Säureadditionssalze werden erfindungsgemäss hergestellt, indem man

a) das racemische α-[(Methylamino)methyl]-9,10-äthanoanthracen-9(10H)-äthanol auftrennt und das S-(+)-α-[(Methylamino)methyl]-9,10-äthanoanthracen-9(10H)-äthanol, gegebenenfalls in Form eines Säureadditionssalzes, isoliert, oder

b) eine Verbindung der Formel II,

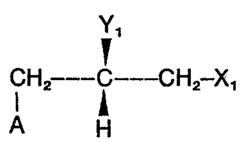

(II),

mit einer Verbindung der Formel III

$$X_2-CH_3 \quad (III)$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-rest steht, oder

c) in einer Verbindung der Formel IV,

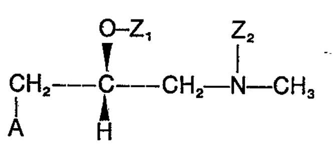

(IV)

in welcher mindestens eines der Symbole $Z_1$ und $Z_2$ einen abspaltbaren Rest und das andere gegebenenfalls Wasserstoff, oder $Z_1$ und $Z_2$ zusammen einen zweiwertigen, abspaltbaren Rest bedeuten, und A für den 9,10-Äthanoanthracen-9(10H)-yl-rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das R-α-[(Methylamino)-methyl]-9(10H)-anthracenäthanol Äthylen anlagert, oder

f) eine Verbindung der allgemeinen Formel V

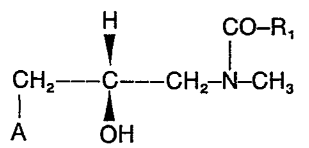

(V)

in welcher $R_1$ einen gegebenenfalls substituierten Kohlenwasserstoffrest oder einen gegebenenfalls substituierten heterocyclischen Rest und A den 9,10-Äthanoanthracen-9(10H)-yl-rest bedeutet, mit einer starken sauerstoffhaltigen anorganischen oder organischen Säure oder einem Halogenid einer solchen umsetzt und das entstandene Zwischenprodukt hydrolysiert, und gewünschtenfalls das so erhaltene S-(+)-α-[(Methylamino)methyl]-9,10-äthanoanthracen-9(10H)-äthanol in ein Säureadditionssalz überführt und/oder aus einem erhaltenen Säureadditionssalz die Base freisetzt.

Die Auftrennung und Isolierung gemäss Verfahren a) erfolgt in an sich bekannter Weise. Beispielsweise kann man das Racemat mit salzbildenden optisch aktiven Säuren, wie organischen Carbon- oder Sulfonsäuren, z.B. die (D)- und (L)-Formen von Weinsäure, Bis-0,0'-(p-toluoyl)-weinsäure, Apfelsäure, Mandelsäure, Camphersulfonsäure, Chinasäure, Milchsäure, Glutaminsäure oder Asparaginsäure in Säureadditionssalze überführen. Die erhaltenen Gemische der entsprechenden Salze können aufgrund von physikalisch-chemischen Unterschieden, z.B. der Löslichkeit oder der Kristallisationsfähigkeit, in die diastereoisomeren Salze aufgetrennt und gegebenenfalls die optisch aktive (+)-Form aus dem Salz freigesetzt werden.

Aus dem Racemat kann man auch durch fraktioniertes Kristallisieren aus einem geeigneten Lösungsmittel, gegebenenfalls auch aus einem optisch aktiven Lösungsmittel, oder durch Chromatographie, insbesondere Dünnschichtchromatographie, an einem optisch aktiven Trägermaterial, die (+)-Form abtrennen.

Eine reaktionsfähige veresterte Hydroxygruppe in einer Verbindung der Formel II oder III ist vor allem eine mit einer starken organischen oder anorganischen Säure, besonders mit einer Halogenwasserstoffsäure, wie Chlor-, Brom- oder Jodwasserstoffsäure, oder mit einer Arylsulfonsäure, wie einer durch niedere Alkyl- oder Alkoxyreste, z.B. die oben genannten, oder durch Halogenatome, wie Chlor- oder Bromatome, ein-, zwei- oder mehrfach substituierten Benzolsulfonsäure, z.B. p-Toluolsulfonsäure oder p-Brombenzolsulfonsäure, oder einer Niederalkansulfonsäure, z.B. Methansulfonsäure, veresterte Hydroxygruppe, oder, besonders als $X_2$, auch eine durch Schwefelsäure bzw. Methylschwefelsäure veresterte Hydroxygruppe. $X_1$ kann auch zusammen mit $Y_1$ als Epoxybrücke vorliegen.

Die Umsetzung gemäss b) erfolgt in üblicher Weise, vorzugsweise in Anwesenheit eines Lösungsmittels und gegebenenfalls in Gegenwart eines Kondensationsmittels, z.B. eines basischen Kondensationsmittels, vorzugsweise bei erhöhter Temperatur und gegebenenfalls im geschlossenen Gefäss unter Druck. Ein basisches Kondensationsmittel ist z.B. ein Alkalihydroxid oder -carbonat, z.B. Natriumhydroxid oder Kaliumcarbonat, oder ein tertiäres Amin, z.B. Triäthylamin oder Pyridin.

In den Ausgangsstoffen der allgemeinen Formel IV für das Verfahren c) sind abspaltbare Reste $Z_1$ und $Z_2$ ebenso wie durch $Z_1$ und $Z_2$ zusammen gebildete zweiwertige abspaltbare Reste, beispielsweise durch Solvolyse, insbesondere Hydrolyse, oder durch Reduktion, z.B. Hydrogenolyse, abspaltbare Reste.

Als durch Solvolyse, insbesondere Hydrolyse, abspaltbare Reste $Z_1$ und $Z_2$ kommen z.B. Acylreste, wie Alkanoylreste, vor allem gegebenenfalls halogenierte, z.B. fluorierte Niederalkanoylreste, wie der Acetylrest bzw. der Trifluoracetylrest, weiter z.B. Aroyl- und Arylniederalkanoylreste, wie der Benzoyl- bzw. Phenylacetylrest, oder Acylreste von Kohlensäurehalbestern, z. B. Niederalkoxycarbonylreste, wie der Methoxycarbonyl-, Äthoxycarbonyl- oder Tert.butoxycarbonylrest, oder Aralkoxycarbonylreste, wie der Benzyloxycarbonylrest, sowie z.B. auch Silylreste, wie der Trimethylsilylrest, in Betracht.

Ein durch $Z_1$ und $Z_2$ gebildeter zweiwertiger Rest ist beispielsweise ein geminal zweiwertiger Kohlenwasserstoffrest, insbesondere ein Niederalkylidenrest, wie der Methylen-, Äthyliden- oder 1-Methyläthylidenrest(Isopropyl)-idenrest, oder ein Aralkylidenrest wie der Benzylidenrest, ferner z.B. eine Phosphorylidengruppe, insbesondere eine Niederalkoxyphosphorylidengruppe, wie die Methoxy- oder Äthoxyphosphorylidengruppe.

Die hydrolytische Abspaltung von $Z_1$ und/oder $Z_2$ erfolgt mit hydrolysierenden Mitteln, beispielsweise in Gegenwart von sauren Mitteln, wie z.B. verdünnten Mineralsäuren, wie Schwefelsäure oder Halogenwasserstoffsäuren, besonders Salzsäure, oder bei Acylresten vorzugsweise in Gegenwart von basischen Mitteln, z.B. Alkalihydroxiden, wie Natriumhydroxid, in geeigneten organischen oder organischwässrigen Lösungsmitteln, z.B. in gegebenenfalls mit Wasser verdünnten Niederalkanolen, in der Kälte, z.B. Raumtemperatur, oder vorzugsweise unter Erwärmen. Die hydrolytische Abspaltung eines durch $Z_1$ und $Z_2$ gebildeten zweiwertigen Restes kann analog erfolgen.

Durch Reduktion abspaltbare Reste $Z_1$ und $Z_2$ sind beispielsweise 1-Arylniederalkylreste, wie der Benzylrest, oder 1-Arylniederalkoxycarbonylreste, wie der Benzyloxycarbonylrest, welche beispielsweise durch Hydrogenolyse, z.B. durch Reduktion mit katalytisch aktiviertem Wasserstoff, wie Wasserstoff in Gegenwart eines Hydrierungskatalysators, wie eines Palladium- oder Platinkatalysators, abgespalten werden können. Ebenfalls durch Hydrogenolyse können durch $Z_1$ und $Z_2$ zusammen gebildete Aralkylidenreste, wie der Benzylidenrest, abgespalten werden. $Z_1$ oder $Z_2$ kann aber auch ein 2-Halogen-alkoxycarbonylrest sein, wie z.B. der 2,2,2-Trichloräthoxycarbonylrest oder der 2-Jodäthoxycarbonylrest, welche durch Reduktion abgespalten werden können. Zur Reduktion kommt vor allem die metallische Reduktion (sogenannter nascierender Wasserstoff) in Betracht, wie z.B. die Einwirkung von Metall bzw. Metallegierungen, wie auch Amalgamen, vorzugsweise in Gegenwart von Wasserstoff abgebenden Mitteln, wie Carbonsäuren, Alkoholen oder Wasser. Vor allem verwendet man Zink oder Zinklegierungen in Essigsäure. Ferner kommen auch Chrom(II)-verbindungen, wie Chrom(II)-chlorid oder Chrom(II)-acetat, in Betracht. $Z_2$ kann auch eine Arylsulfonylgruppe, wie die Toluolsulfonylgruppe, sein, die in üblicher Weise durch Reduktion mit nascierendem Wasserstoff, z.B. durch ein Alkylimetall, wie Lithium oder Natrium, in flüssigem Ammoniak abgespalten werden kann. Die Abspaltung einer Arylsulfonylgruppe kann auch mit einem Hydrid, z.B. einem der nachstehend im Zusammenhang mit dem Verfahren c) genannten einfachen oder komplexen Hydride, vorzugsweise Lithiumaluminiumhydrid, zweckmässig in Gegenwart eines inerten Lösungsmittels, wie eines ätherartigen organischen Lösungsmittels, z.B. Tetrahydrofuran, vorgenommen werden.

Ausgangsstoffe für das Verfahren d) mit einer Doppelbindung zwischen dem Stickstoffatom und einem benachbarten Kohlenstoffatom sind das S-α-[(Methylenamino)-methyl]-9,10-äthanoanthracen-9(10H)-äthanol und das S-α-[(Methylamino)-methyl]-9,10-äthanoanthracen-9(10H)-äthanol. Bei den in Nachbarstellung zum Stickstoffatom durch Hydroxy substituierten Verbindungen handelt es sich um das S-α-[(Hydroxymethylamino)methyl]-9,10-äthanoanthracen-9(10H)-äthanol und das S-1-(Methylamino)-9,10-äthanoanthracen-9(10H)-propan-1,2-diol. Die Reduktion der vorgenannten 4 Verbindungen kann in üblicher Weise, vorzugsweise mittels eines einfachen oder komplexen Hydrids, z.B. eines Borans, oder eines Dileichtmetallhydrids, z.B. eines Alkalimetall-erdmetallhydrids, wie Natriumborhydrid oder Lithiumaluminiumhydrid, oder eines Alkoxyaluminium- oder Alkoxyborhydrids, z.B. eines der weiter unten genannten, erfolgen.

Es ist aber auch möglich, die Reduktion als Hydrierung mit Wasserstoff in Gegenwart eines Katalysators, wie eines Platin-, Palladium- oder

Nickelkatalysators, oder eines homogenen Katalysators, z.B. einer komplexen Rhodiumverbindung, wie eines Rhodium-chlor-triphenyl-phospinkomplexes, zu vollziehen.

Ist ein dem Stickstoffatom benachbartes Kohlenstoffatom durch einen Oxorest substituiert, so handelt es sich bei den entsprechenden Ausgangsstoffen einerseits um das S-N-Methyl-9,10-äthanoanthracen-9(10H)-lactamid und anderseits um das N-[3-(9,10-Äthanoanthracen-9(10H)-yl)-2(S)-hydroxypropyl]-formamid sowie am Stickstoffatom durch den gleichen Rest substituierte Carbaminsäure-niederalkylester, wie den Methyl- und den Äthylester. Deren Reduktion kann nach den üblichen Verfahren der Amidreduktion, beispielsweise mittels eines einfachen oder komplexen Hydrids, wie eines Borans, z.B. Diboran, oder eines komplexen Dileichtmetallhydrids, insbesondere eines Alkalimetallaluminiumhydrids, wie Lithium- oder Natrium-aluminiumhydrid in einem ätherartigen Lösungsmittel, wie Diäthyläther oder Tetrahydrofuran, oder mittels eines Alkalimetallalkoxyaluminiumhydrids oder -borhydrids, z.B. Natriumdibutoxyaluminiumhydrid oder Natriumtrimethoxyborhydrid, oder eines Erdalkalimetallaluminiumhydrids, wie Magnesium-aluminiumhydrid, oder mittels Natriumborhydrid in einem tertiären Amin, wie Pyridin oder Triäthylamin, oder mittels Aluminiumhydrid-Aluminiumchlorid erfolgen.

Die Einführung des 9,10-Äthanorestes gemäss e) kann in üblicher Weise erfolgen, z.B. durch Umsetzung des entsprechenden Anthracenderivates mit Äthylen nach der Methode von Diels-Alder, vorteilhaft in einem geeigneten Lösungsmittel, wie einem aromatischen Kohlenwasserstoff, z.B. Toluol, und bei erhöhter Temperatur, wie z.B. von 50 bis 250 °C, und/oder unter Druck, wie z.B. bei 2 bis 150 at.

In den Ausgangsstoffen der allgemeinen Formel V für das Verfahren f) ist ein gegebenenfalls substituierter Kohlenwasserstoffrest $R_1$, z.B. Niederalkyl, wie Äthyl, Propyl, Isopropyl, Butyl oder Tert.butyl und vor allem Methyl, weiter z.B. Phenylniederalkyl, wie Benzyl oder 2-Phenyläthyl, oder Phenyl, wobei in diesen oder anderen Resten $R_1$ als Substituenten, z.B. Halogen bis Atomnummer 35, Niederalkyl, z.B. Methyl, Niederalkoxy, z.B. Methoxy oder Aryloxy, z.B. Phenoxy, vorliegen können. Als heterocyclischer Rest ist $R_1$ z.B. Furyl, wie 2-Furyl, Thienyl, wie 2-Thienyl, oder Pyridinyl, wie 3- oder 4-Pyridinyl,

Die entsprechenden Ausgangsstoffe der Formel V lassen sich aus dem z.B. bei der Auftrennung von racemischem α-[(Methylamino)methyl]-9,10-äthanoanthracen-9(10H)-äthanol als Nebenprodukt anfallenden freien R-(−)-α-[(Methylamino)-methyl]-9,10-äthanoanthracen-9(10H)-äthanol nach üblichen Acylierungsmethoden, insbesondere unter Verwendung von Carbonsäurehalogeniden oder Niederalkylestern oder, insbesondere für die Herstellung der als Ausgangsstoff besonders geeigneten Verbindung mit Methyl als $R_1$, von Anhydriden, wie Acetanhydrid, herstellen.

Als starke, sauerstoffhaltige anorganische oder organischen Säuren kommen im Verfahren f) insbesondere konzentrierte Schwefelsäure oder Phosphorsäure, weiter z.B. starke organische Sulfonsäuren, etwa aliphatische Sulfonsäuren, z.B. Methansulfonsäure, oder aromatische Sulfonsäuren, wie eine gegebenenfalls substituierte Phenylsulfonsäure, etwa 4-Methyl-, 4-Brom-, 4-Nitro- oder 2,4-Dinitrophenylsulfonsäure oder Naphthalinsulfonsäuren, z.B. 1-Naphthalinsulfonsäure; und als deren Halogenide in erster Linie die Chloride oder Bromide wie vor allem Thionylchlorid, weiter z.B. Thionylbromid, Sulfurylchlorid, Chlorsulfonsäure, Phosphortrichlorid, Phosphorpentachlorid, Phosphoroxychlorid oder Methansulfonylchlorid in Betracht. Ferner können auch den genannten Halogeniden mehrwertiger Säuren entsprechende gemischte Esterhalogenide, wie etwa ein Niederalkoxysulfonylhalogenid, z.B. Methoxy- oder Äthoxysulfonylchlorid oder Phosphorsäure-niederalkylesterhalogenide, z.B. Phosphorsäuredimethylester-chlorid verwendet werden.

Die Umsetzungen mit starken Säuren, vor allem konz. Schwefelsäure oder Phosphorsäure, werden in An- oder Abwesenheit von Lösungs- oder Verdünnungsmitteln, wie z.B. Acetanhydrid, bei Temperaturen von etwa −50 bis +200 °C, und die Umsetzungen mit Säurehalogeniden, wie z.B. Thionylchlorid, ebenfalls in An- oder Abwesenheit von Lösungs- oder Verdünnungsmitteln, z.B. Kohlenwasserstoffen oder insbesondere Chlorkohlenwasserstoffen, wie Methylenchlorid, in einem Temperaturbereich von etwa −10 bis +70 °C, vorzugsweise von etwa +10 bis +50 °C durchgeführt. Als Reaktionsprodukte dieser Umsetzungen können 2-R-5-(9,10-äthynoantracen-9(10H)-yl)-4,5-dihydro-3-methyl-oxazolium-salze, deren Anion der zur Umsetzung verwendeten Säure entspricht bzw. im Fall von Umsetzungen mit Säurehalogeniden das entsprechende Halogenion ist, angenommen werden.

Die Hydrolyse der Zwischenprodukte wird in saurem oder basischem Medium durchgeführt. Geeignete saure Mittel sind z.B. wässrige Säuren, etwa wässrige Mineralsäuren, z.B. wässrige Salzsäure, Schwefelsäure oder Phosphorsäure. Die saure Hydrolyse wird in einem Temperaturbereich von 0 bis +120 °C, zweckmässigerweise bei +10 bis +50 °C durchgeführt. Als basische Medien sind z.B. wässrige Laugen, etwa die der Alkalien oder Erdalkalien wie Natriumhydroxid, oder Kaliumhydroxid, oder die Hydroxide des Calciums oder Magnesiums geeignet, wobei die genannten Reagentien vorteilhafterweise bei erhöhter Temperatur, etwa in einem Bereich von 50 bis 150 °C eingesetzt werden.

Die Hydrolyse kann auch stufenweise durchgeführt werden, indem man ein Zwischenprodukt gegebenenfalls über die entsprechende freie Base als Zwischenstufe, in wässrigem Medium zu der entsprechenden N-Acylverbindung der allgemeinen Formel V mit umgekehrter sterischer Konfiguration wie die des jeweils verwendeten Ausgangsstoffes der Formel V hydrolysiert, und anschliessend diese Verbindung zu einer der Formel I entsprechenden Verbindung hydrolysiert.

Das Verfahren gemäss f) kann vorteilhafterweise auch so durchgeführt werden, dass man einen unmittelbar vorher hergestellten Ausgangsstoff der allgemeinen Formel V, ohne diesen in reiner Form zu isolieren, im gleichen Reaktionsansatz mit einer geeigneten Säure bzw. einem Halogenid einer solchen umsetzt und das erhaltene Zwischenprodukt ebenfalls ohne weitere Reinigung der Hydrolyse unterwirft.

Die für die Verfahren b) bis e) benötigten optisch aktiven Ausgangsstoffe lassen sich entweder durch Auftrennung von bekannten racemischen, insbesondere basischen Ausgangsstoffen in an sich bekannter Weise, oder analog zu den für die Herstellung des bekannten Racemates benötigten, racemischen Ausgangsstoffen unter Verwendung von optisch aktiven statt racemischen Vorläuferverbindungen herstellen.

Säureadditionssalze, insbesondere pharmazeutisch annehmbare Säureadditionssalze der Verbindung der Formel I, z.B. die weiter oben genannten, können in üblicher Weise hergestellt werden. Beispielsweise versetzt man eine Lösung der Base in einem organischen Lösungsmittel, wie z.B. Methylenchlorid, Äthylacetat, Äthanol oder Isopropanol, mit der als Salzkomponente gewünschten Säure oder einer Lösung derselben im gleichen oder andern organischen Lösungsmitteln, wie Äthylacetat oder Diäthyläther, und filtriert, nötigenfalls nach Abkühlen oder Einengen oder nach Zufügen eines Lösungsmittels mit schlechterem Lösungsvermögen für Salze, wie z.B. Diäthyläther, das ausgefallene Salz ab.

Die vorliegende Erfindung betrifft zudem die Verbindung der Formel I und deren pharmazeutisch annehmbare Säureadditionssalze zur Verwendung als Medikamente, insbesondere als Antidepressiva, z.B. zur Anwendung bei den weiter oben genannten Indikationen, sowie ihre Verwendung zur Herstellung von pharmazeutischen, insbesondere antidepressiv wirksamen Präparaten.

Die Dosierung der Verbindung der Formel I und ihrer pharmazeutisch annehmbaren Säureadditionsalze bei Warmblütern hängt von der Spezies, dem Körpergewicht und Alter und vom individuellen Zustand, sowie von der Applikationsweise ab. Die pro Tag verabreichten Dosen liegen zwischen etwa 0,05 und 3 mg/kg, vorzugsweise zwischen etwa 0,08 und 1,5 mg/kg Körpergewicht. Durchschnittlich wird einem Warmblüter von etwa 70 kg Körpergewicht eine Tagesdosis von etwa 10 bis 150 mg, vorzugsweise von etwa 30 bis 75 mg Wirkstoff verabreicht.

Die vorliegende Erfindung betrifft ebenfalls pharmezeutische Präparate, welche die Verbindung der Formel I oder pharmazeutisch annehmbare Säureadditionssalze dieser Verbindung enthalten. Bei den erfindungsgemässen pharmazeutischen Präparaten handelt es sich insbesondere um solche zur enteralen, wie oralen oder rektalen, sowie zur paranteralen Verabreichung, die den pharmakologischen Wirkstoff allein oder vorzugsweise zusammen mit mindestens einem pharmazeutisch annehmbaren Trägermaterial enthalten. Solche Präparate enthalten den Wirkstoff, d.h. die Verbindung der Formel I oder ein pharmazeutisch annehmbares Säureadditionssalz derselben, in einer für die Verabreichung der obengenannten täglichen Dosen in einer oder mehreren, vorzugsweise drei, Einzeldosen geeigneten Menge und Konzentration.

Erfindungsgemäss anwendbare pharmazeutischen Zusammensetzungen in Doseneinheitsformen, wie Dragées, Tabletten, Kapseln, Suppositorien oder Ampullen, enthalten als Wirkstoff pro Doseneinheit vorzugsweise 2,5–50 mg, insbesondere 5–25 mg, der Verbindung der Formel I oder vorzugsweise eines pharmazeutisch annehmbaren Säureadditionssalzes dieser Base zusammen mit mindestens einem pharmazeutischen Trägerstoff.

Doseneinheitsformen für die perorale Anwendung enthalten als Wirkstoff vorzugsweise zwischen 1 und 50% der Verbindung der Formel I oder eines pharmazeutisch annehmbaren Säureadditionssalzes derselben. Zu ihrer Herstellung kombiniert man den Wirkstoff, z.B. mit festen, pulverförmigen Trägerstoffen, wie Lactose, Saccharose, Sorbit, Mannit; Stärken, wie Kartoffelstärke, Maisstärke oder Amylopektin, ferner Laminariapulver oder Citruspulpenpulver; Cellulosederivaten oder Gelatine, gegebenenfalls unter Zusatz von Gleitmitteln, wie Magnesium- oder Calciumstearat oder Polyäthylenglykolen, zu Tabletten oder zu Dragée-Kernen. Die Dragée-Kerne überzieht man beispielsweise mit konzentrierten Zuckerlösungen, welche z.B. noch arabischen Gummi, Talk und/oder Titandioxid enthalten können, oder mit einem Lack, der in leichtflüssigen organischen Lösungsmitteln oder Lösungsmittelgemischen gelöst ist. Diesen Überzügen können Farbstoffe zugefügt werden, z.B. zur Kennzeichnung verschiedener Wirkstoffdosen.

Als weitere orale Doseneinheitsformen eignen sich Steckkapseln aus Gelatine sowie weiche, geschlossene Kapseln aus Gelatine und einem Weichmacher, wie Glycerin. Die Steckkapseln enthalten den Wirkstoff vorzugsweise als Graunulat, z.B. in Mischung mit Füllstoffen, wie Maisstärke, und/oder Gleitmitteln, wie Talk oder Magnesiumstearat, und gegebenenfalls Stabilisatoren, wie Natriummetabisulfit ($Na_2S_2O_5$) oder Ascorbinsäure. In weichen Kapseln ist der Wirkstoff vorzugsweise in geeigneten Flüssigkeiten, wie flüssigen Polyäthylenglykolen, gelöst oder suspendiert, wobei ebenfalls Stabilisatoren zugefügt sein können.

Als erfindungsgemässe Doseneinheitsformen für die rektale Anwendung kommen z.B. Suppositorien in Betracht, welche aus einer Kombination eines Wirkstoffes mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner eignen sich auch Gelatine-Rektalkapseln, welche aus einer Kombination des Wirkstoffes mit einer Grundmasse bestehen. Als Grundmasse eignen sich z.B. flüssige Triglyceride, Polyäthylenglykole oder Paraffinkohlenwasserstoffe.

Ampullen zur parenteralen, insbesondere intramuskulären Verabreichung enthalten vorzugsweise ein wasserlösliches, pharmazeutisch annehmbares Salz der Verbindung der Formel I in einer Konzentration von vorzugsweise 0,5–5%, gegebenenfalls zusammen mit geeigneten Stabilisierungsmitteln und Puffersubstanzen, in wässriger Lösung.

Die nachfolgenden Beispiele erläutern die Herstellung der Verbindung der Formel I sowie einiger typischer Doseneinheitsformen, sollen jedoch den Umfang der Erfindung in keiner Weise beschränken.

Beispiel 1

184,8 g (0,63 Mol) racemisches α-[(Methylamino)methyl]-9,10-äthanoanthracen-9(10H)-äthanol und 127,5 g (0,315 Mol) (–)-Bis-0,0′-(p-toluoyl)-L-weinsäure werden in 2500 ml Methanol bei 40° gelöst und anschliessend 24 Stunden bei Raumtemperatur stehengelassen. Das ausgefallene Kristallisat des R-(–)-α-[(Methylamino)-methyl]-9,10-äthanoanthracen-9(10)-äthanol-(–)-bis-0,0′-(p-toluoyl)-L-tartrat- (1:1) wird abgenutscht und zweimal mit je 50 ml eiskaltem Methanol nachgewaschen.

Das Filtrat wird im Wasserstrahlvakuum eingedampft. Der Rückstand wird in 500 ml Methylenchlorid gelöst und diese Lösung dreimal mit je 100 ml 2n Natronlauge und dann noch zweimal mit je 100 ml Wasser ausgeschüttelt. Nach Abdampfen des Methylenchlorids erhält man 132,5 g partiell an S-(+)-α-[(Methylamino)methyl]-9,10-äthanoanthracen-9(10H)-äthanol angereicherte Base. Diese Base (0,452 Mol) und 91,4 g (0,226 Mol) (+)-Bis-0,0′-(p-toluoyl)-D-weinsäure werden in 1800 ml Methanol bei 40° gelöst und dann 24 Stunden bei Raumtemperatur stehengelassen. Das ausgefallene Kristallisat wird abgenutscht, in Methanol gelöst, die erhaltene Lösung auf etwa ein Drittel ihres Volumes eingeengt und wiederum 24 Stunden bei Raumtemperatur stehengelassen. Die ausgeschiedenen Kristalle werden abgenutscht und mit wenig kaltem Methanol nachgewaschen, wobei man das S-(+)-α-[(Methylamino)methyl]-9,10-äthanoanthracen-9(10H)-äthanol-(+)-bis-0,0′-(p-toluoyl)-D-tartrat-(1:1) erhält, das bei 178° unter Zersetzung schmilzt; $[\alpha]_D^{20} = +63°$ (c= 0,774 in Methanol).

Zur Gewinnung der freien Base löst man 4,9 g (0,01 Mol) des obigen Salzes in 50 ml Methylenchlorid, extrahiert die Lösung zweimal mit je 15 ml 1n Natronlauge, wäscht sie hierauf zweimal mit je 15 ml Wasser und dampft sie bei ca. 14 mbar ein. Das zurückbleibende kristallierte S-(+)-α-[(Methylamino)methyl]-9,10-äthanoanthracen-9-(10H)-äthanol schmilzt bei 106–107°, $[\alpha]_D^{20} = +9,5$ (c= 1,06 in Methanol). Gewünschtenfalls kann die Base noch aus Äther umkristallisiert werden.

Zur Herstellung des Hydrochlorids werden 88,4 g (0,18 Mol) S-(+)-α-[(Methylamino)methyl]-9,10-äthanoanthracen-9(10H)-äthanol-(+)-bis-0,0′-(p-toluoyl)-tartrat-(1:1) in 300 ml Methylenchlorid gelöst und unter Rühren bei Raumtemperatur mit einer ätherischen Chlorwasserstofflösung versetzt, bis die überstehenden Dämpfe bleibend Kongopapier blau färben. Dabei kristallisiert S-(+)-α-[(Methylamino)methyl]-9,10-äthanoanthracen-9(10H)-äthanol-hydrochlorid aus. Nach Zugabe von 450 ml Äther wird das Kristallisat abgenutscht und anschliessend einmal aus Äthanol-Methanol umkristallisiert. Das so erhaltene Hydrochlorid schmilzt bei 231–232°; $[\alpha]_D^{20} = +9°$ (c= 2,1 in Methanol).

Beispiel 2

a) Zu einer Lösung von 2,93 g (0,010 Mol) R-(–)-α-[(Methylamino)methyl]-9,10-äthanoanthracen-9(10H)-äthanol in 8 ml Dimethylformamid werden bei 5–10° 1,9 ml Acetanhydrid zugetropft. Die Lösung wird 4 Stunden bei Raumtemperatur gerührt, dann auf 60 ml Wasser gegossen und mit 100 ml Äthylacetat extrahiert. Die Äthylacetatlösung wird mit gesättigter Natriumbicarbonatlösung gewaschen, über Natriumsulfat getrocknet und im Vakuum eingedampft. Das zurückbleibende, rohe R-(–)-α-[(N-Methylacetamido)-methyl]-9,10-äthanoanthracen-9(10H)-äthanol kann direkt weiterverarbeitet werden.

b) Das Rohprodukt von a) (2,5 g) wird in 6 ml Methylenchlorid gelöst. Unter Rühren wird bei 5–10° innerhalb 15 Minuten eine Lösung von 0,77 ml Thionylchlorid in 4 ml Methylenchlorid zugegeben. Die Reaktionslösung wird 2 Stunden bei 20° und dann eine Stunde bei 35° gerührt und anschliessend bei ca. 14 mbar (Wasserstrahlvakuum) zur Trockne eingedampft.

c) Der Eindampfrückstand von b) wird in 10 ml Äthanol gelöst, mit 2,5 ml Wasser und 1,6 g Natriumhydroxid versetzt und 3 Stunden unter Rückfluss gekocht. Dann wird das Reaktionsgemisch bei ca. 14 mbar eingeengt, mit 50 ml Eiswasser versetzt und dann mit 100 ml Äthylacetat extrahiert. Die Äthylacetatlösung wird mit Wasser neutral gewaschen, über Natriumsulfat getrocknet und bei ca. 14 mbar eingedampft, wobei die rohe, invertierte Base zurückbleibt.

Der Rückstand wird in 10 ml Methylenchlorid gelöst und mit ätherischer Chlorwasserstofflösung versetzt, wobei das S-(+)-1-[(Methylamino)methyl]-9,10-äthanoanthracen-9(10H)-äthanol-hydrochlorid auskristallisiert. Die Kristalle werden abgenutscht und aus Isopropanol umkristallisiert. Smp. 229–231°, $[\alpha]_D^{20} = +9° \pm 1°$ (c= 1,6 in Methanol).

Der Ausgangsstoff für Abschnitt a) wird wie folgt erhalten:

60 g des im Beispiel 1 zuerst anfallenden R-(–)-α-[(Methylamino)methyl]-9,10-äthanoanthracen-9(10H)-äthanol-(–)-bis-0,0′-(p-toluoyl)-L-tartrat-(1:1) [Smp. 180° unter Zersetzung, $(\alpha)_D^{20} = -64°$ (c= 1,135 in Methanol)] wird in 500 ml Methylenchlorid gelöst und diese Lösung dreimal mit je 100 ml 2n Natronlauge und dann noch zweimal mit je 100 ml Wasser ausgeschüttelt. Das nach Abdampfen des Methylenchlorid zurückbleibende R-(–)-α-[(Methylamino)methyl]-9,10-äthanoanthra-

cen-9(10H)-äthanol (Smp. 107–108°) kann direkt für die Acetylierung gemäss a) verwendet werden.

Beispiel 3

2,93 g (0,010 Mol) R-(–)-α-[(Methylamino)-methyl]-9,10-äthanoanthracen-9(10H)-äthanol werden portionenweise in 14 ml Acetanhydrid gelöst. Diese Lösung versetzt man unter Rühren mit einer Lösung von 1,75 g 96%iger Schwefelsäure in 6 ml Acetanhydrid und kocht dann das Gemisch 3 Stunden unter Rückfluss. Anschliessend wird die erhaltene Lösung bei ca. 14 mbar eingeengt und das zurückbleibende, durch N-Acetylierung und Cyclisierung entstandene Reaktionsprodukt in 30 ml 1n Schwefelsäure aufgenommen und 2 Stunden unter Rückfluss gekocht. Dann werden 50 g Eis zugegeben und das erhaltene Gemisch mit wässriger Ammoniaklösung auf pH 9 eingestellt und zweimal mit je 50 ml Äthylacetat extrahiert. Die vereinigten organischen Phasen werden über Natriumsulfat getrocknet und zur Trockne eingedampft. Die zurückbleibende rohe Base wird in 10 ml Methylenchlorid gelöst und mit ätherischer Chlorwasserstofflösung versetzt, wobei das S-(+)-α-[(Methylamino)methyl]-9,10-äthanoanthracen-9(10H)-äthanol-hydrochlorid auskristallisiert. Es wird abgenutscht und aus Isopropanol umkristallisiert; Smp. 228–230°.

Beispiel 4

3,9 g des gemäss a) erhaltenen, rohen 5(S)-[(9,10-Äthanoanthracen-9(10H)-yl)methyl]-3-methyl-oxazolidin werden mit 60 ml 2n Salzsäure 3 Stunden auf 90° erwärmt. Anschliessend gibt man 5n Natronlauge bis zur alkalischen Reaktion zu, extrahiert mit Methylenchlorid und dampft die organische Phase ein. Das zurückbleibende, rohe S-α-(+)-[(Methylamino)methyl )-9,10-äthanoanthracen-9(10H)-äthanol wird in 10 ml Äthanol gelöst und 1 ml einer 10%igen äthanolischen Chlorwasserstofflösung zugefügt. Durch Versetzen mit Äther wird das Hydrochlorid der obigen Base zur Kristallisation gebracht. Das kristallisierte Hydrochlorid wird abgenutscht, gewünschtenfalls analog Beispiel 1 oder 2 weiter gereinigt.

Der Ausgangstoff kann wie folgt hergestellt werden:

a) 20,0 g S-α-(Aminomethyl)-9,10-äthanoanthracen-9(10H)-äthanol (aus der entsprechenden, in der US-PS 4 017 542, Beispiel 1 beschriebenen racemischen Verbindung vom Smp. 176–177° z. B. ähnlich vorliegendem Beispiel 1 erhältlich) werden in einem Gemisch vom 10 ml 35%iger wässriger Formaldehydlösung und 150 ml Ameisensäure eine Stunde auf 95° erhitzt. Das Reaktionsgemisch wird im Vakuum eingedampft, der Rückstand durch Zugabe von 2n Natronlauge alkalisch gestellt und mit Methylenchlorid extrahiert. Die organische Phase wird eingedampft, wobei das 5(S)-[(9,10-äthanoanthracen-9(10H)-yl)methyl]-3-methyl-oxazolidin zurückbleibt.

Beispiel 5

Zu einer Suspension von 0,7 g Lithiumaluminiumhydrid in 20 ml Tetrahydrofuran gibt man eine Lösung von 1,5 g N-[3-(9,10-Äthanoanthracen-9(10H)-yl)-2(S)-hydroxy-propyl]-formamid in 20 ml Tetrahydrofuran und kocht das Gemisch 4 Stunden unter Rückfluss. Anschliessend wird es abgekühlt und mit 1,4 ml Wasser, dann 1,4 ml 15%iger Natronlauge und nochmals 5 ml Wasser versetzt. Den ausgefallenen Niederschlag filtriert man ab, dampft das Filtrat ein und löst den Rückstand in 2n Essigsäure. Die saure Lösung wird mit Äther gewaschen, hierauf mit 10%iger Natronlauge bis zur alkalischen Reaktion versetzt und mit Methylenchlorid extrahiert. Das Lösungsmittel wird abgedampft und das zurückbleibende, rohe S-(+)-α-[(Methylamino)methyl]-9,10-äthanoanthracen-9(10H)-äthanol analog Beispiel 1 oder 2 in das Hydrochlorid vom Smp. 231–232 übergeführt.

Das als Ausgangsmaterial verwendete, substituierte Formamid kann wie folgt hergestellt werden:

a) 5 g (S)-α-(Aminomethyl)-9,10-äthanoanthracen-9(10H)-äthanol (vgl. Beispiel 4a) werden in 75 ml Ameisensäureäthylester 2 Stunden unter Rückfluss gekocht. Die erkaltete Lösung wird bei ca. 14 mbar zur Trockne eingedampft. Der Rückstand wird in 75 ml Methylenchlorid gelöst, diese Lösung mit 40 ml 1n Salzsäure gewaschen, über Natriumsulfat getrocknet und wiederum bei ca. 14 mbar zur Trockene eingedampft. Das zurückbleibende N-[3-(9,10-Äthanoanthracen-9(10H)-yl)-2(S)-hydroxy-propyl]-formamid kann direkt für die Reduktion verwendet werden.

Beispiel 6

Eine Lösung von 10 g S-α-[(Methylamino)methyl]-anthracen-9(10H)-äthanol in 200 ml Benzol wird im Autoklav mit Äthylen unter einem Druck von 70 at. 6 Stunden auf 70° erwärmt. Anschliessend extrahiert man mit 200 ml 2n Salzsäure. Der saure Extrakt wird alkalisch gestellt und mit Methylenchlorid extrahiert. Die organische Phase wird eingedampft und das zurückbleibende, rohe S-α-(+)-[(Methyl-amino)methyl]-9,10-äthanoanthracen-9(10H)-äthanol analog Beispiel 1 in sein Hydrochlorid übergeführt.

Beispiel 7

a) 100 g S-(+)-α-[(Methylamino)methyl]-9,10-äthanoanthracen-9(10H)-äthanol-hydrochlorid werden mit 202 g Lactose und 195 g Kartoffelstärke vermischt, die Mischung mit einer alkoholischen Lösung von 10 g Stearinsäure befeuchtet und durch ein Sieb granuliert. Nach dem Trocknen mischt man 200 g Kartoffelstärke, 250 g Talk, 3,0 g Magnesiumstearat und 40 g kolloidales Siliciumdioxid zu und presst die Mischung zu 10 000 Tabletten von je 100 mg Gewicht und 10 mg Wirkstoffgehalt, die gewünschtenfalls mit Teilkerben zur feineren Anpassung der Dosierung versehen sein können.

b) Aus 50 g S-(+)-α-[(Methylamino)methyl]-9,10-äthanoanthracen-9(10H)-äthanol-hydrochlorid, 228,40 g Lactose und der alkoholischen Lösung von 7,5 g Stearinsäure stellt man ein Granulat her, das man nach dem Trocknen mit 56,60 g kolloidalem Siliciumdioxid, 200 g Talk, 20 g Kartoffelstärke und 2,50 g Magnesiumstearat mischt und zu 10 000 Dragée-Kernen presst. Diese werden anschliessend mit einem konzentrierten Sirup aus 417,3 g krist. Saccharose, 6 g Schellack, 10 g arabischem Gummi, 0,2 g Farbstoff und 1,5 g Titandioxid überzogen und getrocknet. Die erhaltenen Dragées wiegen je 120 mg und enthalten je 5 mg Wirkstoff.

c) Um 1000 Kapseln mit je 10 mg Wirkstoffgehalt herzustellen, mischt man 10 g S-(+)-α-[(Methylamino)methyl]-9,10-äthanoanthracen-9-(10H)-äthanol-hydrochlorid mit 263 g Lactose, befeuchtet die Mischung gleichmässig mit einer wässrigen Lösung von 2 g Gelatine und granuliert sie durch ein geeignetes Sieb (z.B. Sieb III nach Ph.Helv.V). Das Granulat mischt man mit 10 g getrockneter Maisstärke und 15 g Talk und füllt es gleichmässig in 1000 Hartgelatinekapseln der Grösse 1.

d) Man bereitet eine Suppositoriengrundmasse aus 2,0 g S-(+)-α-[(Methylamino)methyl]-9,10-äthanoanthracen-9(10H)-äthanol-hydrochlorid und 168,0 g Adeps solidus und giesst damit 100 Suppositorien mit je 20 mg Wirkstoffgehalt.

e) Eine Lösung von 25 g S-(+)-α-[(Methylamino)methyl]-9,10-äthanoanthracen-9(10H)-äthanol-hydrochlorid in einem Liter Wasser wird in 1000 Ampullen abgefüllt und sterilisiert. Eine Ampulle enthält eine 2,5%ige Lösung von 25 mg Wirkstoff.

**Patentansprüche für die Vertragsstaaten: BE, CH, DE, FR, GB, IT, LU, NL, SE**

1. S-(+)-α-[(Methylamino)methyl]-9,10-äthanoanthracen-9(10H)-äthanol der Formel I

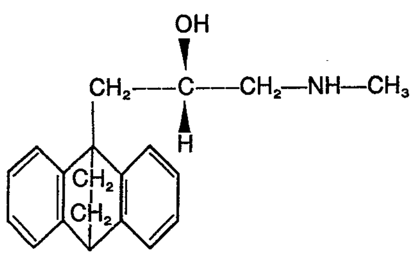

(I)

und seine Säureadditionssalze.

2. Die pharmazeutisch annehmbaren Säureadditionssalze der Verbindung der im Anspruch 1 angegebenen Formel I.

3. Das Hydrochlorid der Verbindung der im Anspruch 1 angegebenen Formel I.

4. Verfahren zur Herstellung des S-(+)-α-[(Methylamino)methyl]-9,10-äthanoanthracen-9-(10H)-äthanol der im Anspruch 1 angegebenen Formel I und seiner Säureadditionssalze, dadurch gekennzeichnet, dass man

a) das racemische α-[(Methylamino)methyl]-9,10-äthanoanthracen-9(10H)-äthanol auftrennt und das S-(+)-α-[(Methylamino)methyl]-9,10-äthanoanthracen-9(10H)-äthanol, gegebenenfalls in Form eines Säureadditionssalzes, isoliert, oder

b) eine Verbindung der Formel II,

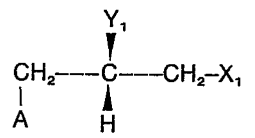

(II),

mit einer Verbindung der Formel III

$$X_2—CH_3 \quad (III)$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-Rest steht, oder

c) in einer Verbindung der Formel IV,

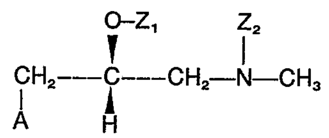

(IV)

in welcher mindestens eines der Symbole $Z_1$ und $Z_2$ einen abspaltbaren Rest und das andere gegebenenfalls Wasserstoff, oder $Z_1$ und $Z_2$ zusammen einen zweiwertigen, abspaltbaren Rest bedeuten, und A für den 9,10-Äthanoanthracen-9(10H)-yl-Rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das S-α-[(Methylamino)methyl]-9(10H)-anthracen-äthanol Äthylen anlagert, oder

f) eine Verbindung der allgemeinen Formel V

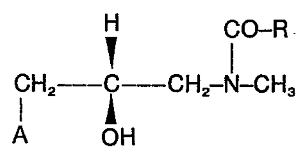

(V)

in welcher R einen gegebenenfalls substituierten Kohlenwasserstoffrest oder einen gegebenenfalls substituierten heterocyclischen Rest und A den 9,10-Äthanoanthracen-9(10H)-yl-Rest bedeutet, mit einer starken sauerstoffhaltigen anorganischen oder organischen Säure oder einem Halogenid einer solchen umsetzt und das entstandene Zwischenprodukt hydrolysiert, und gewünschtenfalls das so erhaltene S-(+)-α-[(Methylamino)methyl]-9,10-äthanoanthracen-9(10H)-äthanol in ein Säureadditionssalz überführt und/oder aus einem erhaltenen Säureadditionssalz die Base freisetzt.

5. Pharmazeutische Präparate, gekennzeichnet durch einen Gehalt an der Verbindung der im

Anspruch 1 angegebenen Formel I oder einem pharmazeutisch annehmbaren Säureadditionssalz derselben, und mindestens einem pharmazeutischen Trägerstoff.

6. Pharmazeutische Präparate gemäss Anspruch 5 in Doseneinheitsform, gekennzeichnet durch einen Gehalt pro Doseneinheit von 2,5 bis 50 mg der Verbindung der in Anspruch 1 angegebenen Formel I oder eines pharmazeutisch annehmbaren Säureadditionssalzes derselben.

7. Pharmazeutische Präparate gemäss Anspruch 5 in Doseneinheitsform, gekennzeichnet durch einen Gehalt pro Doseneinheit von 5 bis 25 mg der Verbindung der in Anspruch 1 angegebenen Formel I oder eines pharmazeutisch annehmbaren Säureadditionssalzes derselben.

8. Pharmazeutische Präparate gemäss einem der Ansprüche 5, 6 und 7, gekennzeichnet durch einen Gehalt an dem Hydrochlorid der Verbindung der in Anspruch 1 angegebenen Formel I.

9. Die Verbindung der im Anspruch 1 angegebenen Formel I oder pharmazeutisch annehmbare Säureadditionssalze derselben zur Anwendung als Antidepressiva.

10. Verwendung der Verbindung der im Anspruch 1 angegebenen Formel I oder eines pharmazeutisch annehmbaren Säureadditionssalzes derselben zur Herstellung von antidepressiv wirksamen pharmazeutischen Präparaten.

**Patentansprüche für den Vertragsstaat: AT**

1. Verfahren zur Herstellung des S-(+)-α-[(Methylamino)methyl]-9,10-äthanoanthracen-9(10H)-äthanol der Formel I

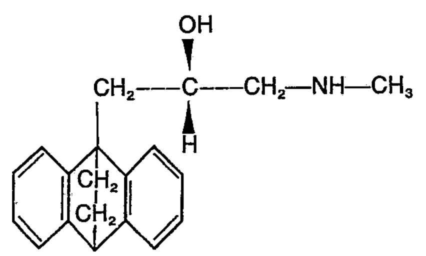

(I)

und seiner Säureadditionssalze, dadurch gekennzeichnet, dass man

a) das racemische α-[(Methylamino)methyl]-9,10-äthanoanthracen-9(10H)-äthanol auftrennt und das S-(+)-α-[(Methylamino)methyl]-9,10-äthanoanthracen-9(10H)-äthanol, gegebenenfalls in Form eines Säureadditionssalzes, isoliert, oder

b) eine Verbindung der Formel II

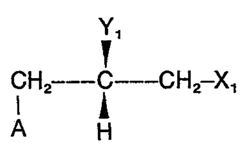

(II),

mit einer Verbindung der Formel III

$$X_2—CH_3 \quad (III)$$

umsetzt, wobei eines der Symbole $X_1$ und $X_2$ die Aminogruppe und das andere eine reaktionsfähige veresterte Hydroxygruppe und $Y_1$ eine freie Hydroxygruppe bedeutet, und $X_1$ auch zusammen mit $Y_1$ eine Epoxygruppe bedeuten kann, und A für den 9,10-Äthanoanthracen-9(10H)-yl-rest steht, oder

c) in einer Verbindung der Formel IV

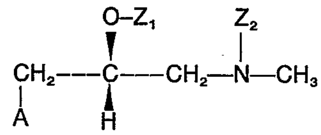

(IV),

in welcher mindestens eines der Symbole $Z_1$ und $Z_2$ einen abspaltbaren Rest und das andere gegebenenfalls Wasserstoff oder $Z_1$ und $Z_2$ zusammen einen zweiwertigen, abspaltbaren Rest bedeuten und A für den 9,10-Äthanoanthracen-9(10H)-yl-Rest steht, den oder die Reste $Z_1$ und/oder $Z_2$ abspaltet, oder

d) eine Verbindung, welche sich von der Verbindung der Formel I nur dadurch unterscheidet, dass in ihr ein dem Stickstoffatom benachbartes Kohlenstoffatom mit letzterem durch eine Doppelbindung verbunden oder durch eine Hydroxygruppe oder durch einen Oxorest, gegebenenfalls zusammen mit Niederalkoxy, substituiert ist, reduziert, oder

e) an das S-α-[(Methylamino)methyl]-9(10H)-anthracen-äthanol Äthylen anlagert, und gewünschtenfalls das so erhaltene S-(+)-α-[(Methylamino)methyl]-9,10-äthanoanthracen-9(10H)-äthanol in ein Säureadditionssalz überführt oder aus einem erhaltenen Säureadditionssalz die Base freisetzt.

2. Abänderung des Verfahrens gemäss Anspruch 1, dadurch gekennzeichnet, dass man eine Verbindung der allgemeinen Formel V

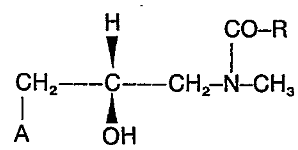

(V),

in welcher R einen gegebenenfalls substituierten Kohlenwasserstoffrest oder einen gegebenenfalls substituierten heterocyclischen Rest und A den 9,10-Äthanoanthracen-9(10H)-yl-Rest bedeutet, mit einer starken sauerstoffhaltigen anorganischen oder organischen Säure oder einem Halogenid einer solchen umsetzt und das entstandene Zwischenprodukt hydrolysiert, und gewünschtenfalls das so erhaltene S-(+)-α-[(Methylamino)methyl]-9,10-äthanoanthracen-9(10H)-äthanol in ein Säureadditionssalz überführt oder aus einem erhaltenen Säureadditionssalz die Base freisetzt.

3. Verfahren nach Anspruch 1, dadurch gekennzeichnet, dass man ein pharmazeutisch annehmbares Säureadditionssalz des S-(+)-α-[(Methylamino)methyl]-9,10-äthanoanthracen-9(10H)-äthanol herstellt.

4. Verfahren nach Anspruch 1, dadurch gekennzeichnet, dass man das Hydrochlorid des S-(+)-α-[(Methylamino)methyl]-9,10-äthanoanthracen-9(10H)-äthanol herstellt.

**Claims: (for all designated states: BE, CH, DE, FR, GB, IT, LU, NL, SE**

1. S-(+)-α-[(Methylamino)methyl]-9,10-ethanoanthracene-9(10H)-ethanol of the formula I

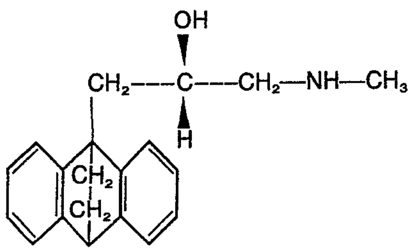

(I)

or an acid addition salt thereof.

2. A pharmaceutically acceptable acid addition salt of the compound of the formula I as indicated in claim 1.

3. The hydrochloride of the compound of the formula I as indicated in claim 1.

4. A process for the production of S-(+)-α-[(methylamino)methyl]-9,10-ethanoanthracene-9-(10H)-ethanol of the formula I as indicated in claim 1, or of an acid addition salt thereof, which process comprises

a) resolving the racemic α-[(methylamino)-methyl]-9,10-ethanoanthracene-9(10H)-ethanol and isolating S-(+)-α-[(methylamino)methyl]-9,10-ethanoanthracene-9(10H)-ethanol, if desired in the form of an acid addition salt, or

b) reacting a compound of the formula II

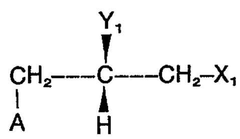

(II),

with a compound of the formula III

$$X_2—CH_3 \qquad (III)$$

wherein one of $X_1$ und $X_2$ is the amino group and the other is a reactive esterified hydroxyl group, and $Y_1$ is a free hydroxyl group, and $X_1$ together with $Y_1$ can also be an epoxy group, and A is the 9,10-ethanoanthracen-9(10H)-yl radical, or

c) in a compound of the formula IV

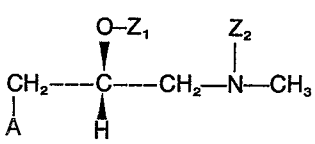

(IV)

in which at least one of $Z_1$ and $Z_2$ is a removable radical and the other may be hydrogen, or $Z_1$ and $Z_2$ together are a divalent removable radical, and A is the 9,10-ethanoanthracen-9(10H)-yl radical, removing $Z_1$ and/or $Z_2$,

d) reducing a compound which differs from the compound of the formula I only in that, in said compound, a carbon atom adjacent to the nitrogen atom is attached to this latter through a double bond or is substituted by a hydroxyl group or an oxo radical, optionally together with lower alkoxy, or

e) adding ethylene to S-α-[(methylamino)-methyl]-9(10H)-anthracene, or

f) reacting a compound of the general formula V

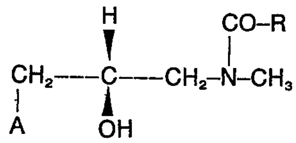

(V)

in which $R_1$ is an unsubstituted or substituted hydrocarbon radical or an unsubstituted or substituted heterocyclic radical, and A is the 9,10-ethanoanthracen-9(10H)-yl radical, with a strong oxygen-containing inorganic or organic acid or with a halide thereof, and hydrolysing the intermediate obtained, and, if desired, converting the resultant S-(+)-α-[(methylamino)methyl]-9,10-ethanoanthracene-9(10H)-ethanol into an acid addition salt and/or liberating the base from a resultant acid addition salt.

5. A pharmaceutical composition containing a compound of the formula I as indicated in claim 1, or a pharmaceutically acceptable acid addition salt thereof, and at least one pharmaceutical carrier.

6. A pharmaceutical composition according to claim 5 in dosage unit form, which contains, per dosage unit, from 2.5 to 50 mg of the compound of the formula I as indicated in claim 1, or of a pharmaceutically acceptable acid addition salt thereof.

7. A pharmaceutical composition according to claim 5 in dosage unit form, which contains, per dosage unit, from 5 to 25 mg of the compound of the formula I as indicated in claim 1, or of a pharmaceutically acceptable acid addition salt thereof.

8. A pharmaceutical composition according to any one of claims 5, 6 or 7, which contains the hydrochloride of the compound of the formula I in claim 1.

9. Use of the compound of the formula I in claim 1, or of a pharmaceutically acceptable acid addition salt thereof, as antidepressant.

10. Use of the compound of the formula I as indicated in claim 1, or of a pharmaceutically acceptable acid addition salt thereof, for the preparation of a pharmaceutical composition having antidepressive properties.

**Claims for the contracting state: AT**

1. A process for the production of S-(+)-α-[(methylamino)methyl]-9,10-ethanoanthracene-9(10H)-ethanol of the formula I as indicated in claim 1, or of an acid addition salt thereof, which process comprises

a) resolving the racemic α-[(methylamino)-methyl]-9,10-ethanoanthracene-9(10H)-ethanol and isolating S-(+)-α-[(methylamino)-methyl]-9,10-ethanoanthracene-9(10H)-ethanol, if desired in the form of an acid addition salt, or

b) reacting a compound of the formula II

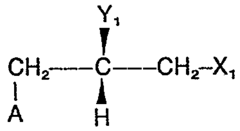
(II),

with a compound of the formula III

$X_2—CH_3$ (III)

wherein one of $X_1$ and $X_2$ is the amino group and the other is a reactive esterified hydroxyl group, and $Y_1$ is a free hydroxyl group, and $X_1$ together with $Y_1$ can also be an epoxy group, and A is the 9,10-ethanoanthracen-9(10H)-yl radical, or

c) in a compound of the formula IV

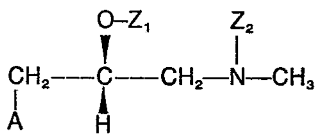
(IV),

in which at least one of $Z_1$ und $Z_2$ is a removable radical and the other may be hydrogen, or $Z_1$ and $Z_2$ together are a divalent removable radical, and A is the 9,10-ethanoanthracen-9(10H)-yl radical, removing $Z_1$ and/or $Z_2$,

d) reducing a compound which differs from the compound of the formula I only in that, in said compound, a carbon atom adjacent to the nitrogen atom is attached to this latter through a double bond or is substituted by a hydroxyl group or an oxo radical, optionally together with lower alkoxy, or

e) adding ethylene to S-α-[(methylamino)-methyl]-9(10H)-anthracene, or hydrolysing the intermediate obtained, and, if desired, converting the resultant S-(+)-α-[(methylamino)methyl]-9,10-ethanoanthracene-9(10H)-ethanol into an acid addition salt and/or liberating the base from a resultant acid addition salt.

2. A modification of the process according to claim 1, which comprises reacting a compound of the general formula V

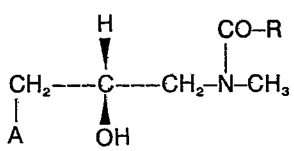
(V),

in which R is an unsubstituted or substituted hydrocarbon radical or an unsubstituted or substituted heterocyclic radical, and A is the 9,10-ethanoanthracen-9(10H)-yl radical, with a strong oxygen-containing inorganic or organic acid or with a halide thereof, and hydrolysing the intermediate obtained, and, if desired, converting the resultant S-(+)-α-[(methylamino)methyl]-9,10-ethanoanthracene-9(10H)-ethanol into an acid addition salt and/or liberating the base from a resultant acid addition salt.

3. A process according to claim 1 for the production of a pharmaceutically acceptable acid addition salt of S-(+)-α-[(methylamino)methyl]-9,10-ethanoanthracene-9(10H)-ethanol.

4. A process according to claim 1 for the production of the hydrochloride of S-(+)-α-[(methylamino)methyl]-9,10-ethanoanthracene-9(10H)-ethanol.

**Revendications (pour tous les Etats contractants: BE, CH, DE, FR, GB, IT, LU, NL, SE**

1. S-(+)-α-[(méthylamino)méthyl]-9,10-éthanoanthracène-9(10H)-éthanol de formule I

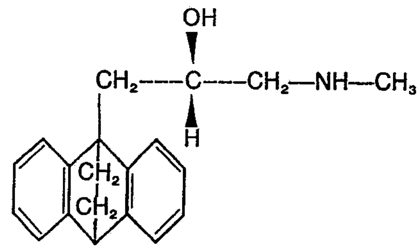
(I)

et ses sels d'addition d'acides.

2. Les sels d'addition d'acides pharmaceutiquement acceptables du composé de formule I donnée dans la revendication 1.

3. Le chlorhydrate du composé de la formula I donnée dans la revendication 1.

4. Procédé de préparation du S-(+)-α-[(méthylamino)méthyl]-9,10-éthanoanthracène-9(10H)-éthanol de la formule I donnée dans la revendication 1 et de ses sels d'addition d'acides, caractérisé en ce que

a) on sépare 1' α-[(méthylamino)méthyl]-9,10-éthanoanthracène-9(10H)-éthanol racémique et on isole le S-(+)-α-[(méthylamino)méthyl]-9,10-éthanoanthracène-0(10H)-éthanol, éventuellement sous la forme d'un sel d'addition d'acide, ou

b) on fait réagir un composé de formule II

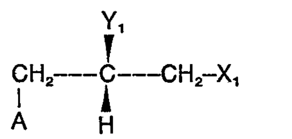
(II),

avec un composé de formule III

$X_2—CH_3$ . (III)

où l'un des symboles $X_1$ et $X_2$ représente le groupe amino et l'autre un groupe hydroxy estérifié réactif et $Y_1$ représente un groupe hydroxy libre, et $X_1$ peut également représenter avec $Y_1$ un groupe époxy, et A représente le radical 9,10-éthanoanthracène-9(10H)-yle, ou

c) dans un composé de formule IV

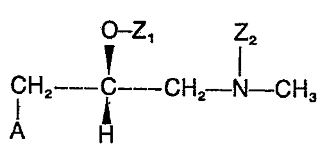
(IV)

où au moins l'un des symboles $Z_1$ et $Z_2$ représente un radical séparable et l'autre éventuellement un hydrogène, ou bien où $Z_1$ et $Z_2$ représentent ensemble un radical séparable bivalent, et A représente le radical 9,10-éthanoanthracène-9(10H)-yle, on sépare le ou les radicaux $Z_1$ et/ou $Z_2$, ou

d) on réduit un composé qui ne se différencie du composé de formule I qu'en ce qu'un atome de carbone voisin de l'atome d'azote y est relié à ce dernier par une double liaison ou y est substitué par un groupe hydroxy ou par un radical oxo, éventuellement avec un alcoxy inférieur, ou

e) on fixe de l'éthylène sur le S-α-[(méthylamino)méthyl]-9(10H)-anthracène-éthanol, ou

f) on fait réagir un composé de formule générale V

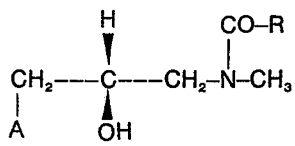

(V)

où R représente un radical hydrocarboné éventuellement substitué ou un radical hétérocyclique éventuellement substitué et A représente le radical 9,10-éthanoanthracène-9(10H)-yle, avec un acide organique ou inorganique fort contenant de l'oxygène ou un halogénure d'un tel acide et on hydrolyse le produit intermédiaire apparu, et si on le désire on transforme le S-(+)-α-[(méthylamino)méthyl]-9-10-éthanoanthracène-9(10H)-éthanol ainsi obtenu en un sel d'addition d'acide et/ou on libère la base à partir d'un sel d'addition d'acide obtenu.

5. Préparations pharmaceutiques, caractérisées en ce qu'elles contiennent le composé de formule I donné dans la revendication 1 ou un de ses sels d'addition d'acide pharmaceutiquement acceptable, et au moins un support pharmaceutique.

6. Préparations pharmaceutiques selon la revendication 5 sous forme posologique unitaire, caractérisées en ce qu'elles contiennent par unité posologique de 2,5 à 50 mg du composé de formule I donné dans la revendication 1 ou d'un de ses sels d'addition d'acides pharmaceutiquement acceptables.

7. Préparations pharmaceutiques selon la revendication 5 sous forme posologique unitaire, caractérisées en ce qu'elles contiennent par unité posologique de 5 à 25 mg du composé de formule I donné dans la revendication 1 ou d'un de ses sels d'addition d'acide pharmaceutiquement acceptables.

8. Préparations pharmaceutiques selon l'une des revendications 5, 6 et 7, caractérisées en ce qu'elles contiennent le chlorhydrate du composé de formule I donnée dans la revendication 1.

9. Composé de formule I donnée dans la revendication 1 ou ses sels d'addition d'acides pharmaceutiquement acceptables aux fins d'application comme anti-dépresseurs.

10. Application du composé de formule I donné dans la revendication 1 ou d'un de ses sels d'addition d'acides pharmaceutiquement acceptables à la préparation de préparations pharmaceutiques à action anti-dépressive.

**Revendications pour l'Etat contractant: AT**

1. Procédé de préparation du S-(+)-α-[(méthylamino)méthyl]-9,10-éthanoanthracène-9(10H)-éthanol de formule I

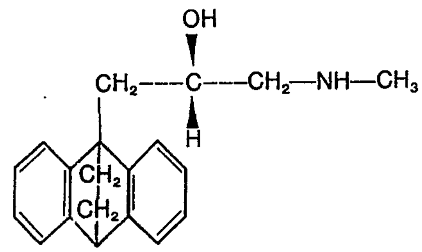

(I)

et de ses sels d'addition d'acides, caractérisé en ce que

a) on sépare l' α-[(méthylamino)méthyl]-9,10-éthanoanthracène-9(10H)-éthanol racémique et on isole le S-(+)-α-[(méthylamino)méthyl]-9,10-éthanoanthracène-9(10H)-éthanol, éventuellement sous la forme d'un sel d'addition d'acide, ou

b) on fait réagir un composé de formule II

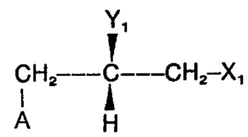

(II)

avec un composé de formule III

$$X_2—CH_3 \qquad \text{(III)}$$

où l'un des symboles $X_1$ et $X_2$ représente le groupe amino et l'autre un groupe hydroxy estérifié réactif et $Y_1$ représente un groupe hydroxy libre, et $X_1$ peut également représenter avec $Y_1$ un groupe époxy, et A représente le radical 9,10-éthanoanthracène-9(10H)-yle, ou

c) dans un composé de formule IV

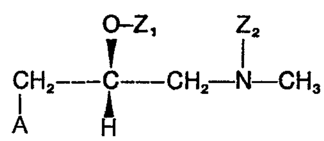

(IV),

où au moins l'un des symboles $Z_1$ et $Z_2$ représente un radical séparable et l'autre éventuellement un hydrogène, ou bien où $Z_1$ et $Z_2$ représentent ensemble un radical bivalent séparable, et A représente un radical 9,10-éthanoanthracène-9(10H)-yle, on sépare le ou les radicaux $Z_1$ et/ou $Z_2$, ou

d) on réduit un composé qui ne se différencie du composé de formule I qu'en ce qu'un atome de carbone voisin de l'atome d'azote y est lié à ce dernier par une double liaison ou y est substitué par un groupe hydroxy ou par un radical oxo, éventuellement avec un alcoxy inférieur, ou

e) on fixe de l'éthylène sur le S-α-[(méthylamino)méthyl]-9(10H)-anthracène-éthanol, et si on le désire on transforme le S-(+)-α-[(méthylamino)méthyl]-9,10-éthanoanthracène-9(10H)-éthanol ainsi obtenu en un sel d'addition d'acide ou on libère la base à partir d'un sel d'addition d'acide obtenu.

2. Modification du procédé selon la revendication 1, caractérisé en ce qu'on fait réagir un composé de formule générale V

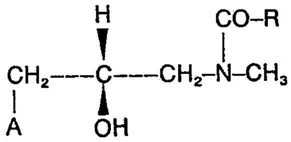

(V) ,

où R représente un radical hydrocarboné éventuellement substitué ou un radical hétérocyclique éventuellement substitué et A représente le radical 9,10-éthanoanthracène-9(10H)-yle, avec un acide inorganique ou organique fort contenant de l'oxygène ou un halogénure d'un tel acide et on hydrolyse le produit intermédiaire obtenu, et si on le désire on transforme le S-(+)-α-[(méthylamino)méthyl]-9,10-éthanoantracène-9(10H)-éthanol ainsi obtenu en un sel d'addition d'acide ou on libère la base à partir d'un sel d'addition d'acide obtenu.

3. Procédé selon la revendication 1, caractérisé en ce qu'on prépare un sel d'addition d'acide pharmaceutiquement acceptable du S-(+)-α-[(méthylamino)méthyl]-9,10-éthanoanthracène-9-(10H)-éthanol.

4. Procédé selon la revendication 1, caractérisé en ce qu'on prépare le chlorhydrate du S-(+)-α-[(méthylamino)méthyl]-9,10-éthanoanthracène-9-(10H)-éthanol.